# EUROPEAN PATENT SPECIFICATION

(11) **EP 3 988 144 B1**
(45) Date of publication and mention of the grant of the patent: **28.08.2024**
(21) Application number: 21202299.0
(22) Date of filing: 13.12.2007
(51) Int. Cl.: A61M 5/142, A61M 39/28, A61M 5/168, A61M 39/08

(54) **ADMINISTRATION SET WITH TWO KEYS**
VERWALTUNGSSATZ MIT ZWEI SCHLÜSSELN
ENSEMBLE D'ADMINISTRATION À DEUX CLÉS

(30) Priority: 25.12.2006 IL 18030506
(43) Date of publication of application: 27.04.2022
(62) Divisional of application: 18196042.8
(73) Proprietor: Caesarea Medical Electronics Ltd., 38900 Caesarea (IL)
(72) Inventor: BARAK, Swi, 38900 Caesarea (IL)
(74) Representative: Harrison IP Limited

(56) References cited:
- EP-A1- 1 938 851
- EP-A2- 1 247 538
- WO-A1-2006/083933
- US-A- 4 798 590
- US-A1- 2005 277 890

## Description

This invention relates to liquid administration and more specially, it is concerned with a liquid flow set useful for administration of liquids to a patient through a flexible tube.

### Background of the invention

Systems for administration of liquids to a patient are widely known. However, a variety of different pumps are available for propelling liquid to a patient, which may differ, among others, by construction and safety of use.

Flow sets for use with a liquid pump must attentively design for safe use. A dedicated tube-segment of the tube must be installed in the pump and the tube must be installed in the right place, tighten, straight and stretched only up to a determined value. The flow set must be full with liquid before using it and should remain full as long as it in used. Moreover, the pumping tube-segment must be replaced by other tube-segment when it losses it's flexibility.

From US 4,798,590 a flow set became known, comprising a drip chamber, a flexible plastic line, having a roller clamp, an inlet connector coupled to an end of a pump chamber, wherein the other end of the pump chamber is coupled to an outlet connector. The outlet connector is coupled through a line having a roller clamp and a Y-site therein a luer.

EP1247538 discloses a disposal flow set comprising a drip chamber or a spike, a first administration tube to administrate liquid from said drip chamber, at least one pumping tube-segment, connected to said first administration tube, a second administration tube in the end of the last of said pumping tube-segments and an anti-free-flow valve installed in the end of said second administration tube.

US2005/277890 discloses a medical delivery system for delivering a medicament or fluid to a patient. The system comprises a disposable element such as a line set associated with a container containing the fluid, an identifier associated with the line set and having identification information associated therewith, and a delivery device configured to engage the line set and deliver the fluid to the patient. The delivery device includes a recognition system that is capable of obtaining the identification information associated with the identifier.

EP2006/083933 discloses a system and method for automatically delivering an infusate to a patient. The system includes an infusion set and an infusion device. A signaling component disposed on an infusion set component identifies an administration protocol for the infusion set. A detection device operatively connected to the infusion device detects the signaling component and identifies the administration protocol. The infusion device is then configured to operate according to the administration protocol.

US4798590 discloses an intravenous infusion pumping system in which an independent and therefore disposable pump set cooperates with a rotor assembly block to produce a peristaltic action conveying a therapeutic solution drawn from a container into a patient. The pump set, which includes a flow line extending between the container and the patient, has a pump chamber interposed therein constituted by a precisely-dimensioned, flexible tube terminating in inlet and outlet connectors. The tube is looped in a block cavity to conform to an arcuate stator wall therein and is subjected to compression by a pair of diametrically-opposed rollers carried by the rotor, the rollers pressing the tube against the wall to occlude the tube and thereby trap the fluid therein. As the rotor turns, the points of occlusion are advanced to propel the fluid toward the outlet. To assure a pumping action in the proper direction, the inlet connector is provided with a flange having a predetermined size which is seated in a matching slot in an inlet socket in the block, the outlet connector having a flange of different size seated in a matching slot in an outlet socket, thereby precluding reversal of the pump chamber. To prevent air embolism, the transparent outlet connector is associated with a light beam detector that senses the presence of air in the flow therethrough to generate a signal which deactivates the pump.

The present invention is concerned with a liquid flow set that includes a number of safety features in order to identify such a flow set by the liquid pump that uses it and also to prevent the pumping tube-segment from over stretching.

### Summary of the invention

According to first aspect of the present invention, there is provided a disposable flow set as hereinafter set forth in Claim 1 of the appended claims.

Features of embodiments of the first aspect of the invention are set forth in the appended Claims 2 to 14.

According to a second aspect of the invention, there is provided a method for installing a disposable flow set of the invention as hereinafter set forth in Claim 15 of the appended claims.

### Brief description of the figures

The invention is herein described, by way of example only, with reference to the accompanying drawings. With specific reference now to the drawings in detail, it is stressed that the particulars shown are by the way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making it apparent to those skilled in the art how the several forms of the invention may be embodied in practice.

### In the figures:

Figure 1 illustrates a flow set with spike (11), an administration tubing (12a), a pumping tube-segment (12b), a stretching-key (13), an identifying-key (14), a second administration tubing (12c) and an anti-free-flow valve (15).
Figure 2 illustrates the way to install a pumping tube segment (12b) in a liquid pump (21).
Figure 3 illustrates a cross section of identifying-key (14).
Figure 4 illustrates a cross section of the stretching-key (13).

### Description of the preferred embodiment

The present invention is a flow set for administration of liquid when using a liquid pump and for either identification by a liquid pump that uses this flow set and prevention of pumping tube-segment over stretching.

Flow sets for use with a liquid pump must attentively be designed for safe use. A dedicated tube-segment of the tube must be installed in the pump and the tube must be installed in the right place, correct direction, tightened, straight and stretched only to a determined degree. The flow set must be full with liquid before using it and should remain full as long as it is in use. Moreover, the pumping tube-segment must be replaced by another tube-segment when it losses it's flexibility.

The flow set of the present invention has a number of features that ensures the use of only this specific flow set in a compatible liquid pump. There is an identifying-key that prevents the insertion of a wrong flow set and enables the liquid pump to determine the presence of the compatible administration set.

In addition, the flow set of the present invention has a number of features that ensure the use of the flow set in a way that prevents pumping tube-segment from over stretching.

The principles and operation of the flow set according to the present invention, and the use of the flow set, may be better understood with reference to the drawings and the accompanying description.

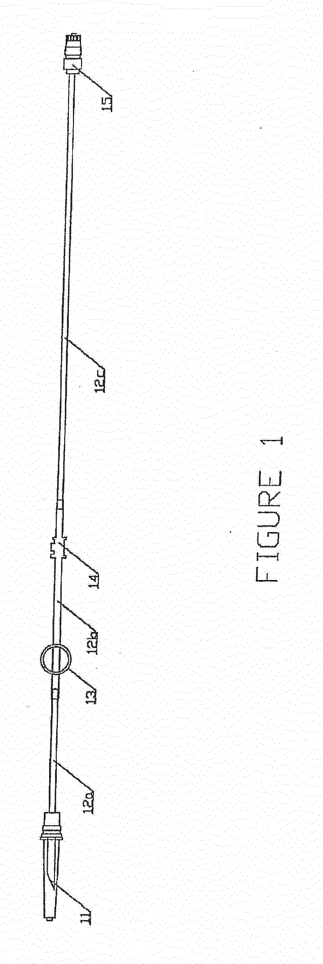

Referring now to the drawings. Figure 1 illustrates a flow set with a spike 11, a first administration tube 12a which administrates the liquid through pumping tube-segments 12b, a second administration tube 12c administrates the pumped liquid to an anti-free-flow valve 15. An identifying-key 14 is installed near one of the pumping tube-segment's ends. Following installation of the identifying-key 14 in a liquid pump 21 the stretching-key 13 is used to install the pumping tube-segment 12b in the liquid pump 21 in a correct position - tightened, straight and stretched up to the determined degree.

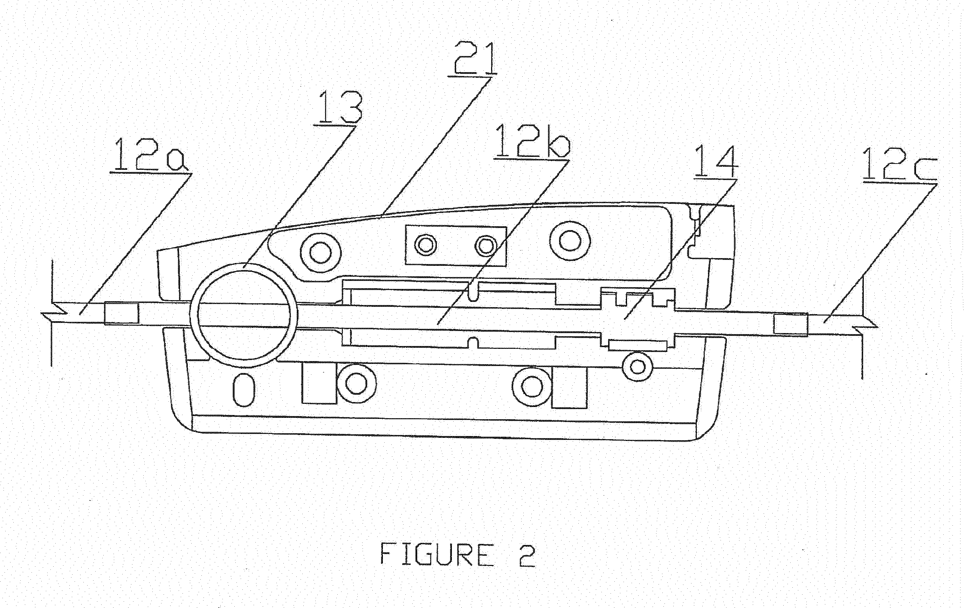

Figure 2 illustrates the way to install a pumping tube-segment 12b in a liquid pump 21. An identifying-key 14 is installed on the pumping tube-segment 12b. The identifying-key 14 is clamped to the tube-segment 12b and includes a number of teeth 14a. Each tooth has a specific width and specific width and specific location, creating a code that enables inserting the tube-segment 12b only to a specific liquid pump 21 that has a set of niches 21a that are arranged in the same code. The identifying-key 14 has also a pressure-plate 34a. This pressure-plate 34a is pressed by a door (not shown) of the liquid pump 21 and presses the pumping tube-segment 12b against a pressure-sensor (not shown) that is installed in the liquid pump 21 behind the identifying-key 14. The identifying-key 14 presents the liquid pump 21 from starting operation unless the pressure-sensor measures a determined pressure. The stretching-key is designed to be installed in a recess 21b of the liquid pump 21. The determined clamping distance and position between the identifying-key 14 and the stretching-key 13 will correspond to the respective distance and position between the identifying-key niches 21a and the stretching-key recess 21b in the liquid pump 21. When both the identifying-key and the stretching-key are installed in the liquid pump 21, the determined distance between their clamping locations on the pumping tube-segment 12b forces the pumping tube-segment 12b to stay tightened, straight and stretched up to a determined degree.

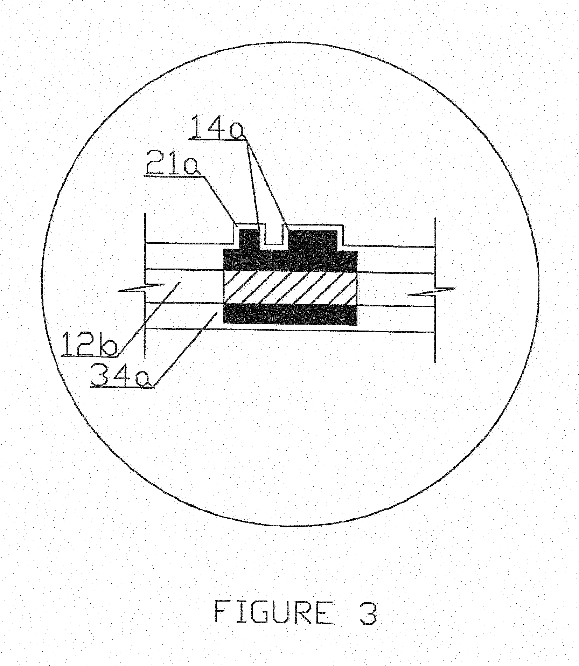

Figure 3 illustrates a cross section of an identifying-key 14. The identifying-key 14 is clamped to the tube-segment 12b near one of its ends. The identifying-key 14 includes a set of teeth 14a to enable the insertion of the flow set only into a compatible liquid pump and presses the pumping tube-segment 12b against a pressure-sensor (not shown) of the liquid pump enabling the pressure-measuring.

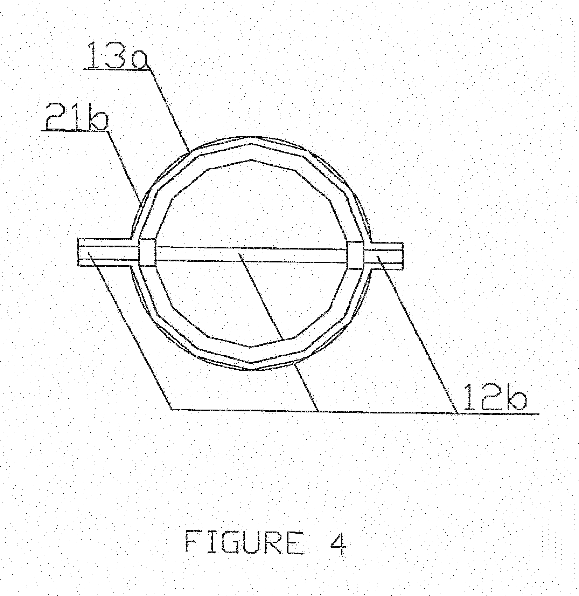

Figure 4 illustrates a cross section of a stretching-key 13. The stretching-key 13 is clamped to the pumping tube-segment 12b near its other end. The stretching-key 13 may be ring-shaped 13a in order to enable an air sensor (not shown) to detect air in the tube-segment part located in said stretching-key center. It also may cause the pumping tube-segment to be placed in front of the air sensor for optimal detection of air in the pumping tube-segment.

Although the invention has been described in conjunction with specific embodiments thereof, it is evident that may alternatives, modifications and variations will be apparent to those skilled in the art, accordingly it is intended to embrace all such alternatives modifications and variations that fall within scope of the appended claims.

## Claims

1. A disposable flow set (10) comprising:
a spike (11);
a first administration tube-segment (12a);
a pumping tube-segment (12b);
a second administration tube-segment (12c);
an anti-free-flow valve (15); and
an identifying-key (14) configured to be clamped on said pumping tube-segment (12b), the identifying-key (14) comprising:
a number of teeth (14a) with a unique combination of location and width, said teeth (14a) being insertable into compatible niches (21a) in a compatible liquid pump (21) so as to prevent insertion of said disposable flow set (10) to a non-compatible liquid pump (21); and
a pressing-plate (34a) to be pressed by a door of the liquid pump (21) in order to allow said pumping tube-segment (12b) to be pressed against a pressure-sensor of said liquid pump (21) when the door of said liquid pump (21) is closed to enable said liquid pump (21) to use the sensed pressure to verify the presence of said disposable flow set (10);
said disposable flow set (10) being **characterized by**:
a stretching-key (13) configured to be clamped on said pumping tube-segment (12b), said stretching-key (13) being disposed on the pumping tube-segment (12b) at a distance from the identifying-key (14) and having a unique pattern (13a) to be inserted into a compatible recess (21b) in a compatible liquid pump (21) to prevent insertion of the disposable flow set (10) in a non-compatible liquid pump (21) and to maintain the pumping tube-segment (12b) under tension, straightened and stretched to a determined degree when inserted into the liquid pump (21).

2. The disposable flow set (10) of claim 1, wherein the distance between the identifying-key (14) and the stretching-key (13) ranges from 45 millimeters to about 47 millimeters.

3. The disposable flow set (10) of either of claims 1 and 2, wherein the stretching-key (13) is ring-shaped.

4. The disposable flow set (10) of any one of claims 1 to 3, wherein the stretching-key (13) is configured to allow detection of air in the pumping tube-segment (12b) by a liquid pump air detector through a hole in the stretching-key (13).

5. The disposable flow set (10) of claim 4, wherein the stretching-key (13) is configured to be disposed in front of the liquid pump air detector when the stretching-key (13) is installed in the liquid pump (21).

6. The disposable flow set (10) of claim 4, wherein the stretching-key (13) is configured to be disposed in back of the liquid pump air detector when the stretching-key (13) is installed in the liquid pump (21).

7. The disposable flow set (10) of any one of claims 1 to 6, wherein the stretching-key (13) is sized and shaped to ensure that following installation of the identifying-key (14) and the stretching-key (13), the pumping tube-segment (12b) is correctly positioned in the liquid pump (21).

8. The disposable flow set (10) of any one of claims 1 to 7, wherein the stretching-key (13) is sized and shaped to ensure that following installation of the identifying-key (14) and the stretching-key (13), the pumping tube-segment (12b) is stretched to a predetermined degree when inserted into the liquid pump (21a).

9. The disposable flow set (10) of any one of claims 1 to 8, wherein the stretching-key (13) is sized and shaped to ensure that following installation of the identifying-key (14) and the stretching-key (13), the pumping tube-segment (12b) is under tension when inserted into the liquid pump (21a).

10. The disposable flow set (10) of any one of claims 1 to 9, wherein the stretching-key (13) is sized and shaped to ensure that following installation of the identifying-key (14) and the stretching-key (13), the pumping tube-segment (12b) is straight when inserted into the liquid pump (21a).

11. The disposable flow set (10) of any one of claims 1 to 10, wherein the stretching-key (13) is configured to center the pumping tube-segment (12b) in front of an air sensor detector of the liquid pump (21) to ensure optimal air detection in the pumping tube-segment (12b).

12. The disposable flow set (10) of any one of claims 1 to 11, wherein the stretching-key (13) is configured to clamp to a pumping tube-segment (12b) having an inside diameter between 2 millimeters and 4 millimeters.

13. The disposable flow set (10) of any one of claims 1 to 12, wherein the identifying-key (14) is configured to clamp to a pumping tube-segment (12b) having an inside diameter between 2 millimeters and 4 millimeters.

14. The disposable flow set (10) of any one of claims 1 to 13, further comprising an anti-free-flow valve (15) at a second end of said second administration tube-segment (12c).

15. A method for installing the disposable flow set (10) of any of claims 1 to 14, the method comprising:
inserting said identifying-key (14) clamped to said pumping tube-segment (12b) into said liquid pump (21) so that said teeth (14a) are received by said niches (21a);
inserting said stretching-key (13) into said recess (21b); and
forcing, based on a determined distance between said identifying-key (14) and said stretching-key (13), the pumping tube-segment (12b) to stay tightened, straight and stretched up to a determined degree.

## Patentansprüche

1. Einweg-Fluidset (10), umfassend:
einen Dorn (11);
ein erstes Verabreichungsrohrsegment (12a);
ein Pumprohrsegment (12b);
ein zweites Verabreichungsrohrsegment (12c);
ein Anti-Freistrom-Ventil (15); und
einen Identifikationsschlüssel (14), der dazu konfiguriert ist, an das Pumprohrsegment (12b) geklemmt zu werden, wobei der Identifikationsschlüssel (14) Folgendes umfasst:
eine Anzahl von Zähnen (14a) mit einer einzigartigen Kombination aus Lage und Breite, wobei die Zähne (14a) in kompatible Nischen (21a) in einer kompatiblen Flüssigkeitspumpe (21) einsetzbar sind, um das Einsetzen des Einweg-Fluidsets (10) in eine nicht kompatible Flüssigkeitspumpe (21) zu verhindern; und
eine Druckplatte (34a), die durch eine Tür der Flüssigkeitspumpe (21) zu drücken ist, um zu ermöglichen, dass das Pumprohrsegment (12b) gegen einen Drucksensor der Flüssigkeitspumpe (21) gedrückt wird, wenn die Tür der Flüssigkeitspumpe (21) geschlossen ist, um es der Flüssigkeitspumpe (21) zu ermöglichen, den erfassten Druck zu verwenden, um das Vorhandensein des Einweg-Fluidsets (10) zu verifizieren;
wobei das Einweg-Fluidset (10) durch Folgendes gekennzeichnet ist:
einen Spannschlüssel (13), der dazu konfiguriert ist, an das Pumprohrsegment (12b) geklemmt zu werden, wobei der Spannschlüssel (13) an dem Pumprohrsegment (12b) in einer Entfernung von dem Identifikationsschlüssel (14) angeordnet ist und ein einzigartiges Muster (13a) aufweist, das in eine kompatible Aussparung (21b) in einer kompatiblen Flüssigkeitspumpe (21) einzusetzen ist, um ein Einsetzen des Einweg-Fluidsets (10) in eine nicht kompatible Flüssigkeitspumpe (21) zu verhindern und um das Pumprohrsegment (12b) unter Spannung zu halten und es um ein bestimmtes Maß zu begradigen und zu strecken, wenn es in die Flüssigkeitspumpe (21) eingesetzt wird.

2. Einweg-Fluidset (10) nach Anspruch 1, wobei die Entfernung zwischen dem Identifikationsschlüssel (14) und dem Spannschlüssel (13) im Bereich von 45 Millimetern bis etwa 47 Millimetern liegt.

3. Einweg-Fluidset (10) nach einem von Anspruch 1 und 2, wobei der Spannschlüssel (13) ringförmig ist.

4. Einweg-Fluidset (10) nach einem der Ansprüche 1 bis 3, wobei der Spannschlüssel (13) dazu konfiguriert ist, eine Detektion von Luft in dem Pumprohrsegment (12b) durch einen Flüssigkeitspumpenluftdetektor über ein Loch in dem Spannschlüssel (13) zu ermöglichen.

5. Einweg-Fluidset (10) nach Anspruch 4, wobei der Spannschlüssel (13) dazu konfiguriert ist, vor dem Flüssigkeitspumpenluftdetektor angeordnet zu sein, wenn der Spannschlüssel (13) in der Flüssigkeitspumpe (21) eingebaut ist.

6. Einweg-Fluidset (10) nach Anspruch 4, wobei der Spannschlüssel (13) dazu konfiguriert ist, hinter dem Flüssigkeitspumpenluftdetektor angeordnet zu sein, wenn der Spannschlüssel (13) in der Flüssigkeitspumpe (21) eingebaut ist.

7. Einweg-Fluidset (10) nach einem der Ansprüche 1 bis 6, wobei der Spannschlüssel (13) so bemessen und geformt ist, dass nach dem Einbau des Identifikationsschlüssels (14) und des Spannschlüssels (13) sichergestellt ist, dass das Pumprohrsegment (12b) richtig in der Flüssigkeitspumpe (21) positioniert ist.

8. Einweg-Fluidset (10) nach einem der Ansprüche 1 bis 7, wobei der Spannschlüssel (13) so bemessen und geformt ist, dass sichergestellt ist, dass nach dem Einbau des Identifizierungsschlüssels (14) und des Spannschlüssels (13) das Pumprohrsegment (12b) um ein vorbestimmtes Maß gestreckt wird, wenn es in die Flüssigkeitspumpe (21a) eingesetzt wird.

9. Einweg-Fluidset (10) nach einem der Ansprüche 1 bis 8, wobei der Spannschlüssel (13) so bemessen und geformt ist, dass sichergestellt ist, dass nach dem Einbau des Identifikationsschlüssels (14) und des Spannschlüssels (13) das Pumprohrsegment (12b) unter Spannung steht, wenn es in die Flüssigkeitspumpe (21a) eingesetzt ist.

10. Einweg-Fluidset (10) nach einem der Ansprüche 1 bis 9, wobei der Spannschlüssel (13) so bemessen und geformt ist, dass sichergestellt ist, dass nach dem Einbau des Identifikationsschlüssels (14) und des Spannschlüssels (13) das Pumprohrsegment (12b) gerade ist, wenn es in die Flüssigkeitspumpe (21a) eingesetzt ist.

11. Einweg-Fluidset (10) nach einem der Ansprüche 1 bis 10, wobei der Spannschlüssel (13) dazu konfiguriert ist, das Pumprohrsegment (12b) vor einem Luftsensordetektor der Flüssigkeitspumpe (21) zu zentrieren, um eine optimale Luftdetektion in dem Pumprohrsegment (12b) sicherzustellen.

12. Einweg-Fluidset (10) nach einem der Ansprüche 1 bis 11, wobei der Spannschlüssel (13) dazu konfiguriert ist, an ein Pumprohrsegment (12b) mit einem Innendurchmesser zwischen 2 Millimetern und 4 Millimetern geklemmt zu werden.

13. Einweg-Fluidset (10) nach einem der Ansprüche 1 bis 12, wobei der Identifikationsschlüssel (14) dazu konfiguriert ist, an ein Pumprohrsegment (12b) mit einem Innendurchmesser zwischen 2 Millimetern und 4 Millimetern geklemmt zu werden.

14. Einweg-Fluidset (10) nach einem der Ansprüche 1 bis 13, ferner umfassend ein Anti-Freistrom-Ventil (15) an einem zweiten Ende des zweiten Verabreichungsrohrsegments (12c).

15. Verfahren zum Einbauen des Einweg-Fluidsets (10) nach einem der Ansprüche 1 bis 14, wobei das Verfahren Folgendes umfasst:
Einführen des an das Pumprohrsegment (12b) geklemmten Identifikationsschlüssels (14) in die Flüssigkeitspumpe (21), sodass die Zähne (14a) von den Nischen (21a) aufgenommen werden;
Einführen des Spannschlüssels (13) in die Aussparung (21b); und
Zwingen des Pumprohrsegments (12b) dazu, auf Grundlage einer bestimmten Entfernung zwischen dem Identifikationsschlüssel (14) und dem Spannschlüssel (13) bis zu einem bestimmten Grad straffgezogen, gerade und gedehnt zu bleiben.

## Revendications

1. Ensemble d'écoulement jetable (10) comprenant :
une pointe (11) ;
un premier segment de tube d'administration (12a) ;
un segment de tube de pompage (12b) ;
un second segment de tube d'administration (12c) ;
une soupape anti-écoulement libre (15) ; et
une clé d'identification (14) conçue pour être serrée sur ledit segment de tube de pompage (12b), la clé d'identification (14) comprenant :
un certain nombre de dents (14a) avec une combinaison unique d'emplacement et de largeur, lesdites dents (14a) pouvant être insérées dans des niches (21a) compatibles dans une pompe à liquide (21) compatible de manière à empêcher l'insertion dudit ensemble d'écoulement jetable (10) dans une pompe à liquide (21) non compatible ; et
une plaque de pression (34a) devant être pressée par une porte de la pompe à liquide (21) afin de permettre audit segment de tube de pompage (12b) d'être pressé contre un capteur de pression de ladite pompe à liquide (21) lorsque la porte de ladite pompe à liquide (21) est fermée pour permettre à ladite pompe à liquide (21) d'utiliser la pression détectée pour vérifier la présence dudit ensemble d' écoulement jetable (10) ;
ledit ensemble d'écoulement jetable (10) étant **caractérisé par** :
une clé d'étirage (13) conçue pour être serrée sur ledit segment de tube de pompage (12b), ladite clé d'étirage (13) étant disposée sur le segment de tube de pompage (12b) à distance de la clé d'identification (14) et comportant un motif unique (13a) destiné à être inséré dans un évidement (21b) compatible dans une pompe à liquide (21) compatible pour empêcher l'insertion de l'ensemble d'écoulement jetable (10) dans une pompe à liquide (21) non compatible et pour maintenir le segment de tube de pompage (12b) sous tension, redressé et étiré jusqu'à un degré déterminé lorsqu'il est inséré dans la pompe à liquide (21).

2. Ensemble d'écoulement jetable (10) selon la revendication 1, ladite distance entre la clé d'identification (14) et la clé d'étirage (13) allant de 45 millimètres à environ 47 millimètres.

3. Ensemble d' écoulement jetable (10) selon l'une ou l'autre des revendications 1 et 2, ladite clé d'étirage (13) étant en forme d'anneau.

4. Ensemble d'écoulement jetable (10) selon l'une quelconque des revendications 1 à 3, ladite clé d'étirage (13) étant conçue pour permettre la détection d'air dans le segment de tube de pompage (12b) par un détecteur d'air de pompe à liquide à travers un trou dans la clé d'étirage (13).

5. Ensemble d'écoulement jetable (10) selon la revendication 4, ladite clé d'étirage (13) étant conçue pour être disposée devant le détecteur d'air de pompe à liquide lorsque la clé d'étirage (13) est installée dans la pompe à liquide (21).

6. Ensemble d'écoulement jetable (10) selon la revendication 4, ladite clé d'étirage (13) étant conçue pour être disposée à l'arrière du détecteur d'air de pompe à liquide lorsque la clé d'étirage (13) est installée dans la pompe à liquide (21).

7. Ensemble d'écoulement jetable (10) selon l'une quelconque des revendications 1 à 6, ladite clé d'étirage (13) étant dimensionnée et mise en forme de manière à garantir qu'après installation de la clé d'identification (14) et de la clé d'étirage (13), le segment de tube de pompage (12b) soit correctement positionné dans la pompe à liquide (21).

8. Ensemble d'écoulement jetable (10) selon l'une quelconque des revendications 1 à 7, ladite clé d'étirage (13) étant dimensionnée et mise en forme de manière à garantir qu'après l'installation de la clé d'identification (14) et de la clé d'étirage (13), le segment de tube de pompage (12b) soit étiré jusqu'à un degré prédéfini lorsqu'il est inséré dans la pompe à liquide (21a).

9. Ensemble d'écoulement jetable (10) selon l'une quelconque des revendications 1 à 8, ladite clé d'étirage (13) étant dimensionnée et mise en forme de manière à garantir qu'après installation de la clé d'identification (14) et de la clé d'étirage (13), le segment de tube de pompage (12b) soit sous tension lorsqu'il est inséré dans la pompe à liquide (21a).

10. Ensemble d'écoulement jetable (10) selon l'une quelconque des revendications 1 à 9, ladite clé d'étirage (13) étant dimensionnée et mise en forme de manière à garantir qu'après installation de la clé d'identification (14) et de la clé d'étirage (13), le segment de tube de pompage (12b) soit droit lorsqu'il est inséré dans la pompe à liquide (21a).

11. Ensemble d'écoulement jetable (10) selon l'une quelconque des revendications 1 à 10, ladite clé d'étirage (13) étant conçue pour centrer le segment de tube de pompage (12b) devant un détecteur de capteur d'air de la pompe à liquide (21) de manière à assurer une détection d'air optimale dans le segment de tube de pompage (12b).

12. Ensemble d'écoulement jetable (10) selon l'une quelconque des revendications 1 à 11, ladite clé d'étirage (13) étant conçue pour se serrer sur un segment de tube de pompage (12b) comportant un diamètre interne compris entre 2 millimètres et 4 millimètres.

13. Ensemble d'écoulement jetable (10) selon l'une quelconque des revendications 1 à 12, ladite clé d'identification (14) étant conçue pour se fixer à un segment de tube de pompage (12b) comportant un diamètre interne compris entre 2 millimètres et 4 millimètres.

14. Ensemble d'écoulement jetable (10) selon l'une quelconque des revendications 1 à 13, comprenant en outre une soupape anti-écoulement libre (15) au niveau d'une seconde extrémité dudit second segment de tube d'administration (12c).

15. Procédé d'installation de l'ensemble d'écoulement jetable (10) selon l'une quelconque des revendications 1 à 14, le procédé comprenant :
l'insertion de ladite clé d'identification (14) serrée sur ledit segment de tube de pompage (12b) dans ladite pompe à liquide (21) de sorte que lesdites dents (14a) soient reçues par lesdites niches (21a) ;
l'insertion de ladite clé d'étirage (13) dans ledit évidement (21b) ; et
le fait de contraindre, sur la base de la distance définie entre ladite clé d'identification (14) et ladite clé d'étirage (13), le segment de tube de pompage (12b) à rester serré, droit et étiré jusqu'à un degré déterminé.
